# EUROPEAN PATENT APPLICATION

(11) **EP 1 215 499 A1**
(43) Date of publication of application: **19.06.2002**
(21) Application number: 00960976.9
(22) Date of filing: 13.09.2000
(51) Int. Cl.: G01N 33/569

(54) **METHOD OF SEARCHING FOR SUBSTANCE HAVING ANTI-INFLUENZA VIRUS EFFECT**

(30) Priority: 13.09.1999 JP 25937299
(71) Applicant: Kyorin Pharmaceutical Co., Ltd., Tokyo 101-8311 (JP); Kido, Hiroshi, Tokushima-shi, Tokushima 770-0811 (JP)
(72) Inventor: KIDO, Hiroshi, Tokushima-shi, Tokushima 770-0811 (JP); MURAKAMI, Meiko, Tokushima-shi, Tokushima 770-0045 (JP)
(74) Representative: VOSSIUS & PARTNER
(86) International application number: JP0006255
(87) International publication number: WO0120332

(57) **Abstract**

A method is provided that makes it possible to efficiently find anti-influenza virus substances that alleviate problems accompanying the prior art and act based on a different mechanism than that by which the prior art substances act.

The method for finding substances with anti-influenza virus activity is characterized in that miniplasmin is used as a probe.

## Description

### Technical Field

The present invention relates to a method for finding substances with anti-influenza virus activity.

### Technical Background

There are basically three different approaches that have been taken in developing anti-influenza virus substances.

The first approach is to develop vaccines as anti-influenza virus substances. Vaccines such as inactivated influenza vaccines and live vaccines have been developed thus far.

The second approach is to find and develop channel blockers as anti-influenza virus substances that are targeted to M₂ proteins in ion channels of influenza viruses.

The third approach is to find and develop anti-influenza virus substances that are targeted to sialic acids, which are found on the cell membrane and are known to serve as a receptor for influenza viruses upon infection.

However these approaches are accompanied by the respective disadvantages such as follows.

In the first approach, which is directed to development of vaccines as anti-influenza virus substances, vaccines may not always be effective since surface glycoproteins of influenza virus, which are in many cases recognized as antigens, keep changing every year from one epidemic event to the next.

In regard of the second approach, which intends to find and develop channel blockers that are targeted to M₂ proteins in ion channels of influenza viruses, for example, Amantadine, which has traditionally been known as an effective treatment against Parkinson's disease and has proven to act as an M₂ protein blocker, may exhibit strong side effects on the central nervous system and its use is currently restricted, making the substance inapplicable to all of the patients who suffer from influenza. Therefore, substances obtained through this approach may have the problem associated with the central nervous system.

In regard of the third approach, which intends to find and develop anti-influenza virus substances that are targeted to sialic acids, which are found on the cell membrane and are known to serve as a receptor for influenza viruses upon infection, substances obtained through this approach have yet to be fully exploited though they have been found to be effective. Further, this approach relies on completely different sites of action and mechanism than those that the present invention takes advantage of.

### Disclosure of the Invention

The present invention has been devised to alleviate the above-described problems with the prior art and provides an effective approach for finding anti-influenza virus substances that act based on a different mechanism than that by which the prior art substances act.

During the course of our studies to understand the relationship between miniplasmin, and influenza virus and Sendai virus, the present inventors have found that miniplasmin is a key enzyme involved in the activation of influenza viruses, especially those that can cause acute or chronic pulmonary infection accompanied by infiltration of neutrophils. In other words, the present inventors have discovered that miniplasmin is an enzyme that plays a key role in the activation of influenza viruses or Sendai viruses and, in order for these viruses to become infectious in a human body, it is essential that miniplasmin should transform the viral structure into an active form.

Based on these findings, the present inventors have reached the conclusion that developing a way to find miniplasmin inhibitors, substances that can block the action of miniplasmin to activate influenza virus, will facilitate the search for anti-influenza virus substances for practical medical use.

Accordingly, one aspect of the present invention provides a method for finding anti-influenza virus substances that takes advantage of miniplasmin as a probe.

Another aspect of the present invention provides a method for finding anti-influenza virus substances, in which miniplasmin serving as the probe, and Sendai virus or influenza virus serving as a substrate are allowed to react with each other in the presence of a substance of interest. Subsequently, the amounts of subunits of a precursor of surface glycoproteins of the virus serving as the substrate in the reaction solution are used as indices for determining if the substrate of interest has anti-influenza virus activity.

Another aspect of the present invention provides a method for finding anti-influenza virus substances, in which miniplasmin serving as the probe, and Sendai virus or influenza virus serving as a substrate are allowed to react with each other in the presence of a substance of interest and subsequent to the reaction, the virus serving as the substrate is allowed to infect Mardin Darby canine kidney cells (referred to as MDCK cells, hereinafter) to obtain a cell infection unit as an index for determining if the substrate of interest has anti-influenza virus activity.

The present inventors have observed that miniplasmin, an enzyme principally formed in a local region where a substantial neutrophil infiltration takes place because of a disease condition such as inflammation, is found attached to cell membranes in the local region, and when the cells are infected with influenza viruses, non-infectious influenza or Sendai viruses newly proliferating or budding from the infected cells are transformed, through the action of miniplasmin, into infectious viruses capable of infecting cells in the respiratory tract.

This finding led the present inventors to the idea that, by taking advantage of this characteristic of miniplasmin, a method for finding specific inhibitors of miniplasmin can be established, so that newly discovered drugs through such a method can be used to prevent the progress of influenza infection as represented by various inflammatory responses.

We shall now look into how we made these findings. As shown in Table 1, miniplasmin, produced either by elastase derived from human granulocytes or by elastase derived from a pig pancreas, is significantly more hydrophobic than plasmin and thus is more readily attached to the surfaces of various cell membranes. This observation led the present inventors to suspect significant involvement of the enzyme in the protein cleavage, or transformation of the viruses into their infectious forms.

Influenza viruses and Sendai virus infect respiratory tracts to proliferate. Upon infection, hemagglutinin (HA), a precursor of surface glycoproteins in influenza viruses, must be cleaved into a hemagglutinin 1 (HA₁) subunit and a hemagglutinin 2 (HA₂) subunit, while a fusion protein (F₀), a precursor of surface glycoproteins in Sendai virus, must be cleaved into a fusion protein 1 (F₁) subunit and a fusion protein 2 (F₂) subunit, by host's protease. This is because the viruses are not able to exhibit the ability to fuse cell membranes or the ability to infect cells unless the precursors of the surface glycoproteins are cleaved.

For this reason, we studied influenza viruses and Sendai virus, each representing the viruses of the kind that infects respiratory tracts, to see if miniplasmin was involved in the expression of the membrane-fusion ability and the infectivity of these viruses by being able to partially digest the surface glycoproteins of these viruses. The study revealed that miniplasmin was capable of partially digesting the surface glycoproteins of influenza viruses and Sendai virus, thereby making non-infectious viruses infectious.

The method of the present invention will now be described.

First, a substance of interest that one wants to test for anti-influenza virus activity is added to a system including human miniplasmin and its substrate, for example, Sendai virus or influenza virus, and reactions are allowed to take place. Following the reaction, if influenza virus has been used to serve as the substrate, then the resulting products from the reaction vessel are analyzed for the presence of hemagglutinin 1 (HA₁) and hemagglutinin 2(HA₁) subunits, which result from the cleavage of hemagglutinin (HA), a precursor of surface glycoproteins of influenza viruses. If Sendai virus has been used to serve as the substrate, then the products are analyzed for the presence of fusion protein 1 (F₁) and fusion protein 2 (F₂) subunits, which result from the cleavage of a fusion protein (F₀), a precursor of surface glycoproteins of Sendai virus.

Presence of these subunits indicates that the human miniplasmin has exhibited the intended action, which proves that the substance of interest does not have anti-influenza virus activity, whereas absence of these subunits indicates that the action of human miniplasmin has been inhibited by the substance of interest, thereby proving the anti-influenza virus activity of the substance of interest.

In this manner, substances having anti-influenza virus activity can be found.

Aside from the above-described technique, MDCK cells may be infected with influenza virus and the cell infection unit (CIU) is counted to see if the action of human miniplasmin has been inhibited. In particular, a substance of interest that one wants to test for anti-influenza virus activity is added to a system including human plasmin and influenza virus to serve as the substrate, and reactions are allowed to proceed. Subsequently, influenza virus is collected from the reaction solution and is allowed to infect MDCK cells. The infected cells are detected with an anti-influenza antibody labeled with a fluorescent marker and the CIU is counted. If the CIU value obtained after the viruses have been allowed to react with miniplasmin to be activated in an experiment to which the substance of interest has been added is lower than that obtained in the control that does not include the substance of interest, the substance has been proved to have anti-influenza virus activity. In this manner, it is possible to determine whether if the substance of interest has anti-influenza virus activity.

When synthetic substrates such as those shown in Table 3 are used to serve as indices for finding miniplasmin inhibitors in the method of the invention, unlike the method in which a protein or an actual virus is employed to serve as the substrate, the inhibition rate may vary due to different conformation of each of the synthetic substrates. For this reason, it is preferred to use an actual influenza virus or Sendai virus as the substrate in the present invention.

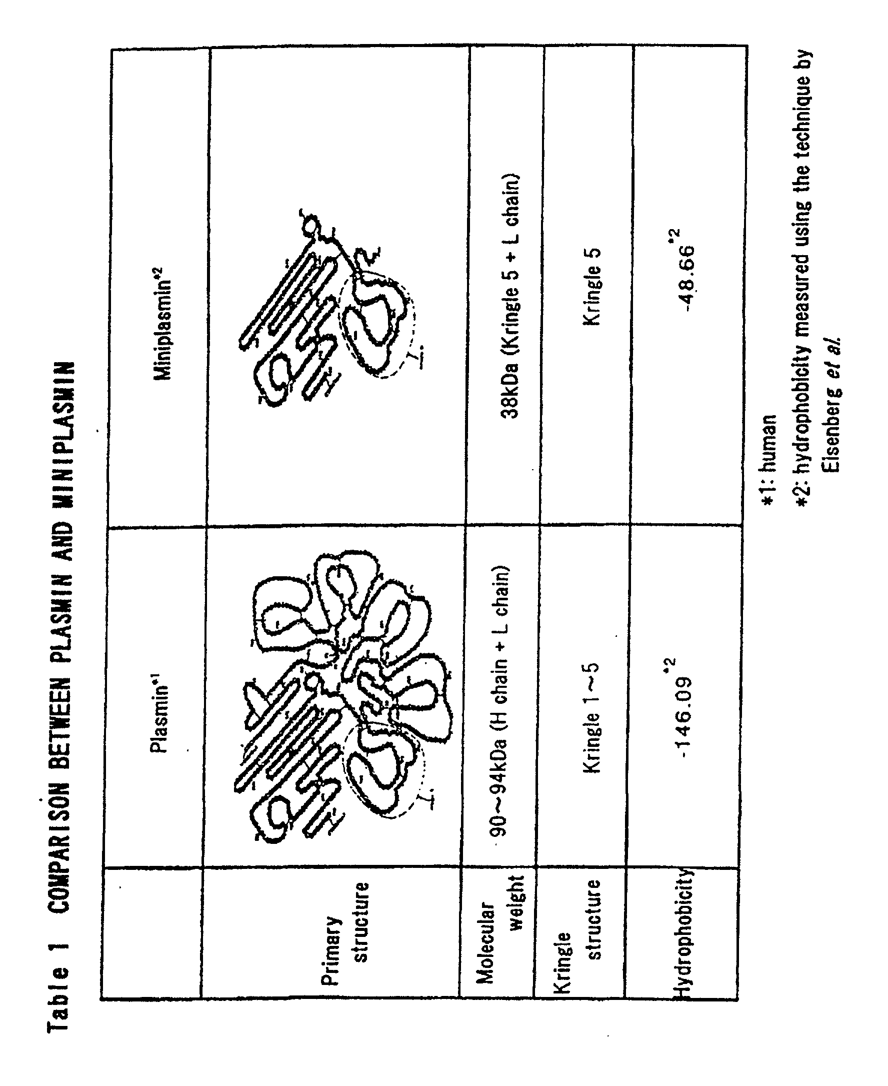

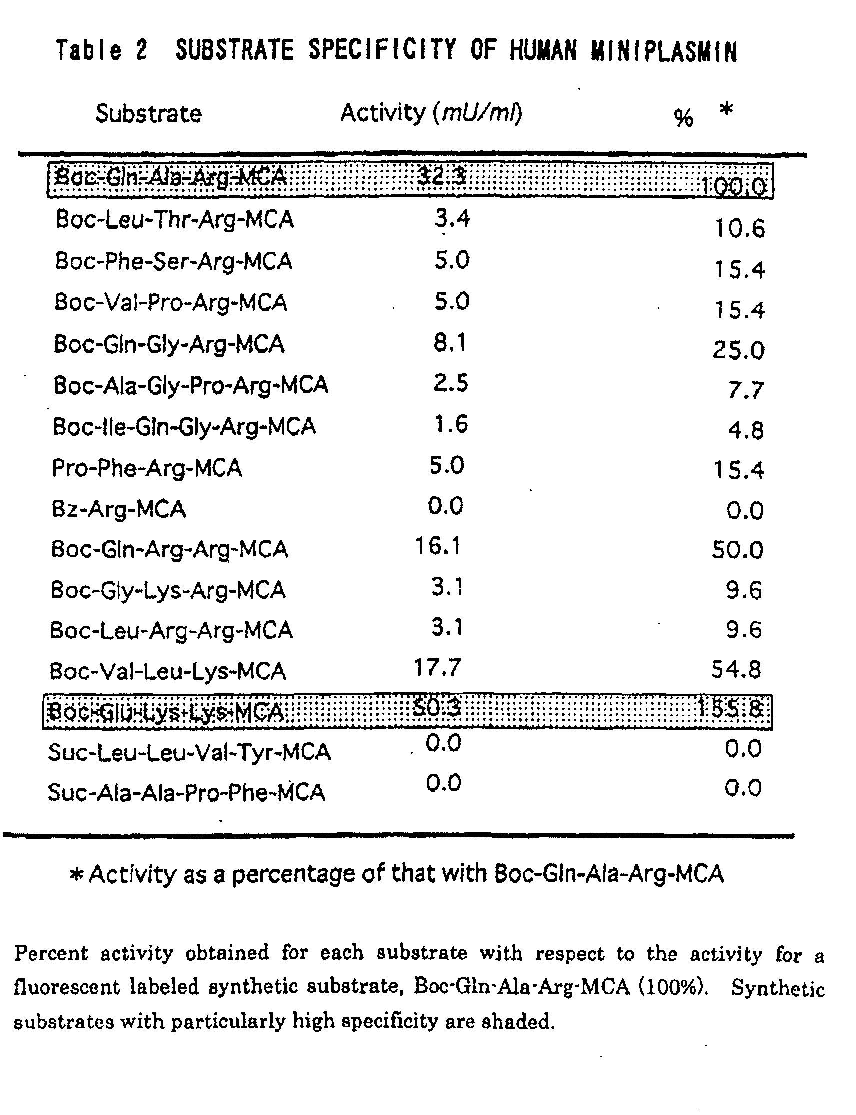

**TABLE 3**

| INHIBITOR SPECIFICITY OF HUMAN MINIPLASMIN | | |
|---|---|---|
| Addition | Final concentration | Relative*activity |
| | | % |
| None | | 100.0 |
| ○ Phenylmethylsulfonyl fluoride | 1mM | 95.1 |
| | 10mM | 29.5 |
| ○ Diisopropylfluorophosphate | 1mM | 65.6 |
| | 10mM | 3.3 |
| ⓞ Aprotinin | 10µM | 0.0 |
| Anti-leuko protease | 10µM | 97.4 |
| ⓞ Leupeptin | 10µM | 18.6 |
| Elastatinal | 10µM | 69.2 |
| ○ Benzamidine | 10µM | 67.6 |
| | 1mM | 22.9 |
| ⓞ Kunitz-type soybean trypsin inhibitor | 10µM | 0.0 |
| Cymostatin | 10µM | 100.0 |
| ⓞ Bowman-Birk soybean trypsin inhibitor | 10µM | 3.7 |
| α₁-Antitrypsin | 10µM | 100.0 |
| E-64 | 10µM | 61.5 |
| Pepstatin A | 10µM | 64.0 |
| Phosphoramidon | 10µM | 100.0 |

| | | |
|---|---|---|
| * Activity as a percentage of that with Boc-Glu-Lys-Lys-MCA | | |

Percent inhibitor specificity obtained for each inhibitor with respect to the activity obtained for a synthetic substrate Boc-Glu-Lys-Lys-MCA (100%), which had the highest specificity toward the purified enzyme. A double circle indicates that the inhibitor exhibited strong inhibition. A circle indicates that the inhibitor exhibited increased inhibition at higher concentrations.

### Examples

### 1. Structure of human miniplasmin

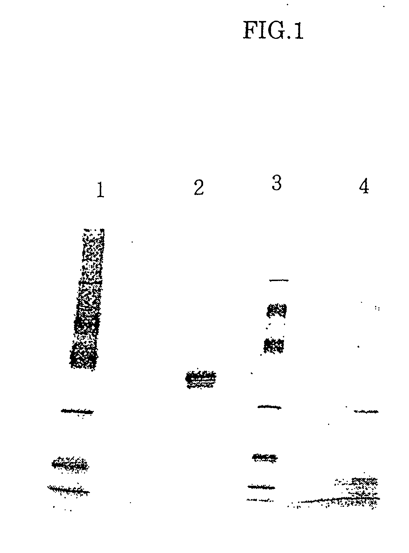

Referring to Fig. 1, a result of SDS-polyacrylamide gel electrophoresis performed on purified human miniplasmin is shown. A single protein band appeared over the range from about 36kDa to 38kDa in the absence of reducing agent, whereas the miniplasmin was separated into two bands at 28kDa and at 12kDa in the presence of reducing agent. This indicates that miniplasmin includes the 28kDa protein and the 12kDa protein that are linked to one another through disulfide bonds. The protein bands at 28kDa and at 12kDa were transferred to a PVDF membrane, which was subjected to an amino acid sequence analysis to determine the amino acid sequence of each protein consisting of about 20 amino acid residues from the N-terminal. It was determined from this analysis that the protein band of 12kDa had the sequence of VVAPPPVVLLPNVETPSEED- and the protein band of 28kDa had the sequence of VVGGCVAHPHSWP WDVSLRY-. Protein bands appeared again at 12kDa and 28kDa when miniplasmin was treated with elastase obtained from human granulocytes. These proteins proved to have precisely the same amino acid sequences as the previously-identified proteins.

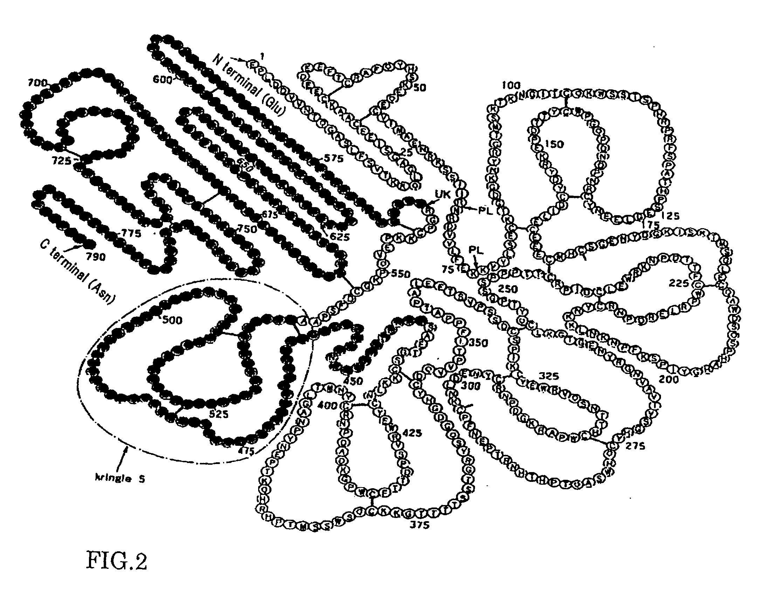

These results suggest that as shown in Fig. 2, the 12kDa protein of human miniplasmin includes a kringle 5 that begins with V⁴⁴¹ and the 28kDa protein includes a microplasmin that begins with V⁵⁶¹.

### 2. Characteristics of human miniplasmin

Miniplasmin obtained in the above-described manner has different characteristics as compared to plasmin. As shown in Table 1, miniplasmin lacks domains of kringles 1 to 4 (angiostatin) that are present on the N-terminal of plasmin and thus has a molecular weight of 38kDa, which is smaller than the molecular weight of plasmin of 94kDa. Miniplasmin also has a significantly higher hydrophobicity than plasmin and thus binds so securely to the surface of a cell membrane that it cannot be solubilized unless surfactants, such as NaCl solution with a concentration of 0.5M or higher or 0.5% Triton X (polyoxyethylene octylether manufactured by Sigma Co., Ltd.), are used.

Microplasmin, which has the same structure as miniplasmin except that it does not have kringle 5 of miniplasmin, becomes highly unstable at substantially neutral pHs and undergoes autolysis in a matter of minutes, resulting in the activity that is reduced by 50% or more from the initial activity. In contrast, the activity of miniplasmin can last as long as a few hours under the same conditions, though unstable.

### 3. Substrate specificity of human miniplasmin

The substrate specificity of human miniplasmin is shown in Table. 2. Among the different trypsin-type proteases, miniplasmin showed the highest cleaving activity toward Boo-Glu-Lys-Lys-MCA, a synthetic substrate for plasmin. Miniplasmin also showed a considerably high cleaving activity toward a group of synthetic substrates, each of which included Glu(Glu)-X-Arg, the same type of amino acid sequence as the one that serves as a recognition site upon cleavage (cleavage motif) and is common among human influenza viruses reported thus far. Miniplasmin cut the bond after Arg.

In contrast, miniplasmin showed substantially no cleaving activity toward Boc-Ile-Gly-Arg-MCA, a synthetic substrate for factor Xa, which is one of the blood clotting factors and is also a protease with the activity similar to that of tripsin. Nor did it toward Bz-Arg-MCA, a synthetic substrate for cathepsin B, which is one of lysosomal enzymes.

### 4. Inhibitor specificity of human miniplasmin

The inhibitor specificity of human miniplasmin is shown in Table 3. Among different protease inhibitors, aprotinin, which was derived from bovine lungs, a Kunitz-type soybean trypsin inhibitor and a Bowman-Birk trypsin inhibitor, each of which was derived from plants, showed strong inhibition against the protease activity of miniplasmin. In contrast, anti-leukoprotease (also known as MPI or SLPI), which inhibits activity of elastase and trypsin, showed substantially no inhibition against the activity of miniplasmin. Benzamizine, diisopropylfluorophosphate, and phenylmethylsulfonyl fluoride, each of which inhibits activity of trypsin, showed strong inhibition when present at high concentrations of 1mM to 10mM.

### 5. Activity of human miniplasmin to activate influenza viruses and Sendai virus

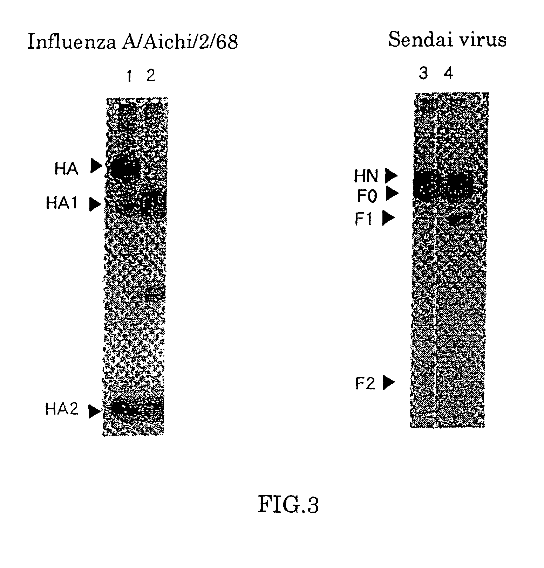

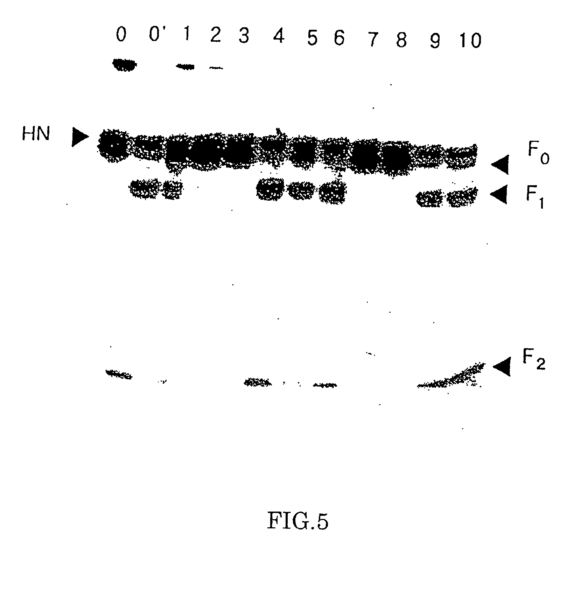

Non-infectious influenza virus and Sendai virus were labeled with [³H]-glucosamine and were treated with miniplasmin (1.5ig) for 15 minutes at 37 C and for 60 minutes at 37 C, respectively. As can be seen in Fig. 3, after the treatment, substantially all of HA of the influenza virus was degraded into HA₁ and HA₂ subunits, while about one-third of F₀ of the Sendai virus was degraded into F₁ and F₂ subunits. F₀ of the Sendai virus was completely degraded into F₁ and F₂ subunits by extending the treatment to as long as 3 hours (Fig. 5).

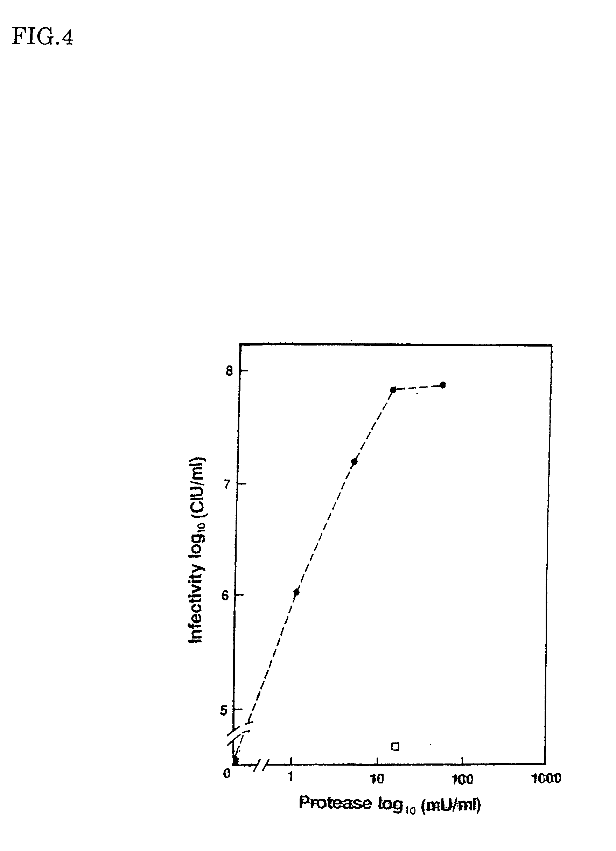

The influenza virus treated with miniplasmin was then allowed to infect MDCK cells, and the cell infection unit (CIU) was measured (Fig. 4). Specifically, non-infectious influenza A/Aichi/2/68(H3N2) strain was treated with different concentrations of miniplasmin at 37 C for 15 minutes in the presence of PBS and was then allowed to infect MDCK cells. The infected cells were detected with a fluorescent-labeled anti-influenza A/Aichi/2/68(H3N2) antibody to count the CIU.

The CIU showed a significant increase as a function of the concentration of miniplasmin and reached a plateau at 10.4mU/ml. The activity of miniplasmin was quantified by defining the enzyme activity that can degrade limol of the synthetic substrate Boc-Glu-Ala-Arg-MCA in 1 minute as 1 unit.

The results indicate that human miniplasmin has activity to transform non-infectious influenza virus and Sendai virus into their infectious forms.

### Brief Description of the Drawings

Fig. 1 is a result of SDS-PAGE performed on human miniplasmin: lane 1, molecular weight marker; lane 2, human miniplasmin (0.2ig); lane 3, molecular weight marker; and lane 4, human miniplasmin (0.2ig).

All lanes were subsequently silver-stained with the lanes 1 and 2 electrophoresed under non-reducing conditions and the lanes 3 and 4 under reducing conditions.

Molecular weight markers: rabbit muscle phospholase B (97.2kDa); BSA (66.4kDa); ovalmin(45.0kDa); carbonic anhydrase (29.0kDa); soybean trypsin inhibitor (20.1kDa); and lysozyme (14.3kDa).

Fig. 2 shows the primary structure of human plasminogen and human miniplasmin: human plasminogen (residue 1-790) and miniplasmin (residue 441-790) are shown.

Fig. 3 is a result of electrophoresis showing the manner in which miniplasmin partially digests an HA protein of influenza A/Aichi/2/68 and an F₀ protein of Sendai virus.
Lane 1: [³H]Glucosamine-labeled Influenza virus A/Aichi/2/68,
Lane 2: [³H]Glucosamine-labeled Influenza virus A/Aichi/2/68 + miniplasmin (1.5ig), incubated for 15min. at 37 C,
Lane 3: [³H]Glucosamine-labeled Sendai virus,
Lane 4: [³H]Glucosamine-labeled Sendai virus + miniplasmin (1.5ig), incubated for 60min. at 37 C.

Fig. 4 is a graph showing the manner in which miniplasmin causes transformation of non-infectious influenza A/Aichi/2/68(H3N2) into its infectious form, and inhibition of the transformation by aprotinin.

Non-infectious influenza virus A/Aichi/2/68(H3N2) was treated either with different concentrations of miniplasmin (solid circles), or with 20mU/ml miniplasmin to which aprotinin has been added to a final concentration of 1iM (hollow squares), for 15 minutes at 37 C and was then allowed to infect MDCK cells. 10 hours after the infection, the infected cells were detected with a fluorescent(FITC)-labeled anti-influenza A/Aichi antibody.

Fig. 5 is a result of electrophoresis showing inhibitor specificity of miniplasmin, in which [³H]-labeled Sendai virus was used as a substrate for the purified enzyme to see the effects of various inhibitors.

| | Final concentration (iM) |
|---|---|
| 0 : [³H]-labeled Sendai virus | |
| 0' : [³H]-labeled Sendai virus + purified enzyme | |
| 1 : [³H]-labeled Sendai virus + purified enzyme + PMSF | 1mM |
| 2 : [³H]-labeled Sendai virus + purified enzyme + DFP | 1mM |
| 3 : [³H]-labeled Sendai virus + purified enzyme + Aprotinin | 1iM |
| 4 : [³H]-labeled Sendai virus + purified enzyme + Anti- leuko protease | 1iM |
| 5 : [³H]-labeled Sendai virus + purified enzyme + Leupeptin | 1iM |
| 6 : [³H]-labeled Sendai virus + purified enzyme + Elastatinal | 1iM |
| 7 : [³H]-labeled Sendai virus + purified enzyme + Benzamidine | 1iM |
| 8 : [³H]-labeled Sendai virus + purified enzyme + Knitz- type soybean trypsin inhibitor | 1iM |
| 9 : [³H]-labeled Sendai virus + purified enzyme + O- phenanthlorin | 1iM |
| 10: [³H]-labeled Sendai virus + purified enzyme + Chymostatin | 1iM |

### Best Modes for Carrying out the Invention

### Reference Example 1 Preparation of human miniplasmin from human plasmin

1mg of human plasmin (Sigma) and 3ig of elastase extracted from a pig pancreas were dissolved in a 50mM Tris-HCl buffer solution (pH8.0). Reactions were allowed to proceed for 3 hours and 15 minutes while being agitated. After the reaction period, elastatinal was added to a final concentration of 50iM. Reactions were allowed to proceed for another 30 minutes at room temperature while being agitated. After the reaction period, a buffer containing NaCl was added to give a final concentration of 50mM Tris-HCl buffer (pH8.0)/0.5M NaCl. The reaction solution was centrifuged for 30 minutes at 14,000xg. Subsequently, the supernatant was loaded onto a soybean trypsin inhibitor sepharose 4B column to allow miniplasmin to be adsorbed onto the medium. The column was thoroughly washed and the adsorbed miniplasmin was then eluted with 50mM glycin-HCl buffer (pH2.8)/0.5M NaCl. The eluate was loaded onto a gel filtration HPLC column (Superdex 200, trade name, manufactured by Amersham Pharmacia Biotech Co., Ltd.) to remove impurities. This resulted in a final product.

### Reference Example 2 Preparation of human miniplasmin from human plasmin

1mg of human plasmin (Sigma) and 3ig of elastase extracted from human granulocytes were dissolved in a 50mM Tris-HCl buffer solution(pH8.0). Reactions were allowed to proceed for 3 hours and 15 minutes while being agitated. After the reaction period, elastatinal was added to a final concentration of 50iM. Reactions were allowed to proceed for another 30 minutes at room temperature while being agitated. After the reaction period, a buffer containing NaCl was added to give a final concentration of 50mM Tris-HCl buffer (pH8.0)/0.5M NaCl. The reaction solution was centrifuged for 30 minutes at 14,000xg. Subsequently, the supernatant was loaded onto a soybean trypsin inhibitor sepharose 4B column to allow miniplasmin to be adsorbed onto the medium. The column was thoroughly washed and the adsorbed miniplasmin was then eluted with 50mM glycin-HCl buffer (pH2.8)/0.5M NaCl. The eluate was loaded onto a gel filtration HPLC column (Superdex 200, trade name, manufactured by Amersham Pharmacia Biotech Co., Ltd.) to remove impurities. This resulted in a final product.

### Example 1

First, [³H]-labeled Sendai virus was treated with miniplasmin (0.1ig) obtained in Reference Example 1 at 37 C for 3 hours. Substantially all F₀ protein underwent partial degradation into F₁ and F₂ subunits (Fig. 5).

Next, 1mM diisopropylfluorophosphate was added to the system and reactions were allowed to proceed for 3 hours at 37 C. The presence of F₁ and F₂ subunits was determined as follows.

After the reaction period, 3iL of a sample buffer solution for electrophoresis, concentrated three-fold (6% SDS, 30% glycerol, 0.2M Tris-HCl buffer, pH6.8), were added to 10iL of the reaction solution. The reaction solution was immediately heat-treated for 5 minutes at 100 C. Subsequently, the sample was subjected to electrophoresis on an SDS-polyacrylamide gel with a 10 to 20% concentration gradient. Each polyacrylamide gel was applied with 30mA for 2 hours. Subsequently, the SDS-polyacrylamide gel was fixed in a fixing solution (50% methanol, 50% acetic acid) for 1 hour and then was treated with a sensitizing solution (Amplify, trade name, manufactured by Amersham Pharmacia Biotech Co., Ltd.) for 20 minutes. After treatment with the sensitizing solution, the SDS-polyacrylamide gel was heat-dried and subjected to autoradiography. Autoradiography was performed on RX-U (trade name, manufactured by FUJI FILM Co., Ltd.), where the sample was exposed at 80 C for three days, followed by development/fixation to detect bands of F₀, F₁ and F₂.

The results showed that transformation of F₀ into F₁ and F₂ was substantially prevented by diisopropylfluorophosphate. Accordingly, diisopropylfluorophosphate has been shown to be an inhibitor of miniplasmin.

### Example 2

1iM of aprotinin was added to the same system as in Example 1 and reactions were allowed to proceed at 37 C for 3 hours. Subsequently, the presence of F₁ and F₂ subunits in the system was determined in the same manner as described in Example 1.

The results indicated that transformation of F₀ into F₁ and F₂ was substantially prevented by aprotinin. Accordingly, aprotinin has been shown to be an inhibitor of miniplasmin.

### Example 3

1iM of banzamidine was added to the same system as in Example 1 and reactions were allowed to proceed at 37 C for 3 hours. Subsequently, the presence of F₁ and F₂ subunits in the system was determined in the same manner as described in Example 1.

The results indicated that transformation of F₀ into F₁ and F₂ was substantially prevented by benzamidine. Accordingly, benzamidine has been shown to be an inhibitor of miniplasmin.

### Example 4

1iM of Kunitz-type soybean trypsin inihibitor was added to the same system as in Example 1 and reactions were allowed to proceed at 37 C for 3 hours. Subsequently, the presence of F₁ and F₂ subunits in the system was determined in the same manner as described in Example 1.

The results indicated that transformation of F₀ into F₁ and F₂ was substantially prevented by Kunitz-type soybean trypsin inihibitor. Accordingly, Kunitz-type soybean trypsin inihibitor has been shown to be an inhibitor of miniplastin.

### Example 5

First, non-infectious influenza A/Aichi/2/68 (H3N2) strain was treated with miniplasmin (944mU/mg) at concentrations of 0, 1.2, 5.2, 10.4, and 52mU/ml in the presence of PBS at 37 C for 15 minutes. Subsequently, the virus was allowed to infect MDCK cells and infected cells were detected with a fluorescent-labeled anti-influenza A/Aichi/2/68 (H3N2) antibody to count the CIUs. The obtained CIUs for the respective concentrations were 0(undetectable), 1x10⁶, 1.3x10⁷, 7.5x10⁷ and 9.6x10⁷ CIU.

Next, 1iM aprotinin was added to the system in which plasmin concentration was sufficiently high (10.4mU/mL) to cause the infectivity plateau, and reactions were allowed to proceed for 15 minutes. The CIU was counted in the same manner as described above.

The CIU after the treatment with 1iM aprotinin was determined to be 3.7x10³CIU. Given the fact that the CIU obtained for the virus activated by miniplasmin in the experiment without any of the substances of interest was 7.5x10⁷, it has been proven that aprotinin has caused the decrease in the CIU and substantially prevented the transformation of non-infectious influenza virus into infectious virus.

### Industrial Applicability

The present invention makes it possible to efficiently find substances with anti-influenza virus activity *in vitro* by employing miniplasmin as a probe.

## Claims

1. A method for finding a substance with anti-influenza virus activity, the method being **characterized in that** miniplasmin is used as a probe.

2. The method for finding a substance with anti-influenza virus activity according to claim 1, **characterized in that** miniplasmin serving as the probe and Sendai virus serving as a substrate are allowed to react with each other in the presence of a substance of interest, such that subsequent to the reaction, amounts of a fusion protein 1 (F₁) subunit and a fusion protein 2 (F₂) subunit of Sendai virus that are present in a reaction solution are used as indices for determining if the substrate of interest has anti-influenza virus activity.

3. The method for finding a substance with anti-influenza virus activity according to claim 1, **characterized in that** miniplasmin serving as the probe and influenza virus serving as a substrate are allowed to react with each other in the presence of a substance of interest, such that subsequent to the reaction, amounts of a hemagglutinin 1(HA₁) subunit and a hemagglutinin 2(HA₂) subunit of influenza virus that are present in a reaction solution are used as indices for determining if the substrate of interest has anti-influenza virus activity.

4. The method for finding a substance with anti-influenza virus activity according to claim 1, **characterized in that** miniplasmin serving as the probe and Sendai virus serving as a substrate are allowed to react with each other in the presence of a substance of interest and subsequent to the reaction, the Sendai virus is allowed to infect MDCK cells to obtain a cell infection unit as an index for determining if the substrate of interest has anti-influenza virus activity.

5. The method for finding a substance with anti-influenza virus activity according to claim 1, **characterized in that** miniplasmin serving as the probe and influenza virus serving as a substrate are allowed to react with each other in the presence of a substance of interest and subsequent to the reaction, influenza virus is allowed to infect MDCK cells to obtain a cell infection unit as an index for determining if the substrate of interest has anti-influenza virus activity.
